# EUROPEAN PATENT SPECIFICATION

(11) **EP 3 569 198 B1**
(45) Date of publication and mention of the grant of the patent: **06.07.2022**
(21) Application number: 19173708.9
(22) Date of filing: 10.05.2019
(51) Int. Cl.: A61F 2/28

(54) **CRANIAL PROSTHESIS**
KRANIALE PROTHESE
PROTHÈSE CRANIENNE

(30) Priority: 14.05.2018 IT 201800005325
(43) Date of publication of application: 20.11.2019
(73) Proprietor: MT Ortho S.r.l., 95025 Aci Sant'Antonio (IT)
(72) Inventor: Sorano, Gaetano, 95039 Trecastagni (CT) (IT); Drago, Roberto, 95029 Viagrande (CT) (IT)
(74) Representative: Lualdi, Lorenzo

(56) References cited:
- WO-A1-2017/042366
- CN-A- 102 274 091
- US-A1- 2006 224 242
- US-A1- 2017 027 629

## Description

### FIELD OF THE INVENTION

The present invention relates to a cranial prosthesis which is implanted in the patient during cranioplasty operations. The closest prior art is document US 2006/224242 A1, which defines the preamble of claim 1.

Various eventualities can lead to the necessity for cranioplasty surgery.

The main ones are post-traumatic craniectomy, for example following bone fragmentation which prevents the reconstruction of the cranial vault, demolitional craniectomy, for example for surgical treatment of tumours, and malformation syndromes that can create bone gaps.

To these, the results of previous surgical operations can be added, which may have led to osteitis and bone resorption around the prosthesis.

In all cases where it is necessary to restore the cranial bone, a prosthesis is used which must reproduce the shape of the natural bone as much as possible.

### STATE OF THE ART

The current surgical technique involves various solutions including that of the manual modelling of a PMMA (polymethylmethacrylate) prosthesis directly in the operating theatre. The limits of this method are the considerable intervention times that increase the costs and risks of infection for the patient, in addition to the limited mechanical and aesthetic properties of the prosthesis due to intraoperative manual modelling.

In recent years, "custom-made" prostheses have been produced, starting from CAT images of the patient. Through appropriate processing, thanks to specific 3D CAD software, the three-dimensional design of the prosthesis can be obtained, which can be produced in different materials.

In the state of the art, the most commonly used biomaterials for the reconstruction of this bone deficiency are hydroxyapatite, polymethylmethacrylate (PMMA), titanium.

Hydroxyapatite is a mineral, having a chemical composition of Ca₅(PO₄)₃(OH), and represents the main component of human bone tissue.

Its introduction as a biomaterial for the reconstruction of bone defects of the skull was initially welcomed with enthusiasm, as it showed osteoconductive properties and it was thought it could be replaced by healthy bone tissue. Hydroxyapatite, however, tends to fracture simply due to the pulsations of the dura mater underlying the bone wall of the skull or following small impacts. When these events occur, tissue fluid enters the prosthesis material, there is propagation of the fracture and finally the complete fragmentation of the device. Furthermore, the use of hydroxyapatite is associated with inflammatory reactions that lead to a reduction in the thickness of the overlying dermis and extrusion of the material.

PMMA (polymethylmethacrylate) is a thermoplastic polymer obtained by polymerization of methyl methacrylate monomer. This biomaterial, in particular, as already mentioned, if modelled in situ, has characteristics that can contribute to increasing the probability of implant failure.

The exothermic polymerization reaction, in fact, generates a temperature higher than 110°C and requires a continuous cooling with cold saline water during the implantation to avoid damage to the surrounding tissues, and the methyl methacrylate monomer, present in the cooled PMMA, is irritating.

These two characteristics are among the causes that contribute to a high failure rate of PMMA implants.

Titanium and its alloys, both in "custom made" systems and in meshes or "nets", allow an excellent possibility of coupling with the bone defect, and allow good aesthetic results to be obtained, with considerable satisfaction for the patient. Furthermore, titanium cranioplasty compared to those produced with other biomaterials, provides an immediately protective reconstruction.

In addition to the drawbacks linked to the material with which cranial prostheses are produced, other drawbacks relate to the form of the prosthesis.

It is in fact known from an analysis of scientific literature on the matter that a high percentage of implant failure of cranial prostheses in hydroxyapatite and PMMA, respectively of 50% and 80%, is a consequence of the very nature of the material used. A much lower value is found instead for titanium cranioplasty, whose failure rate remains well below 10%.

Failures of cranioplasty operations are however also linked to the formation of tissue fluid in the space between the dura mater and the cranioplasty. This accumulation of fluid has a detrimental effect on the cerebral parenchyma and increases the risk of infections. The formation of extradural fluid (which accumulates between the dura mater and the cranial prosthesis) often leads to the necessity of having to remove the implant, with inevitable damage to the patient.

A further drawback which afflicts prostheses of a known type and which has a negative impact on the intervention methods, is linked to the need for preventing the dura mater and the prosthesis from forming a cavity that can cause or at least promote the accumulation of extradural liquid, with the consequences described above.

To avoid this risk, the surgeon sutures the prosthesis to the dura mater in various points in order to obtain the greatest possible adherence of the prosthesis to the dura mater.

For this purpose, the prostheses, in titanium or in other materials, are provided with pre-formed anchoring holes.

In particular, titanium prostheses of the known type are provided for the purpose with a plurality of holes.

### SUMMARY OF THE INVENTION

The main purpose of the present invention is to overcome the drawbacks affecting known solutions, in particular by providing a cranial prosthesis capable of eliminating or at least reducing the risk of failure of the implant.

Within this purpose, the objective of the present invention is to provide a cranial prosthesis capable of decreasing the risk of fluid accumulation between the dura mater and the prosthesis itself.

A further objective of the present invention is to provide a cranial prosthesis which allows the suturing step of the prosthesis itself to the dura mater to be eliminated without jeopardizing the stability and integration of the prosthesis.

These purposes and these and other objectives according to the present invention are achieved by a cranial prosthesis as defined in claim 1.

Further characteristics of the cranial prosthesis according to the present invention are object of the dependent claims.

### LIST OF FIGURES

The characteristics and advantages of the cranial prosthesis for cranioplasty operations according to the present invention will appear more evident from the following detailed description, provided in an exemplifying and non-limiting form, referring to the attached schematic drawings in which:

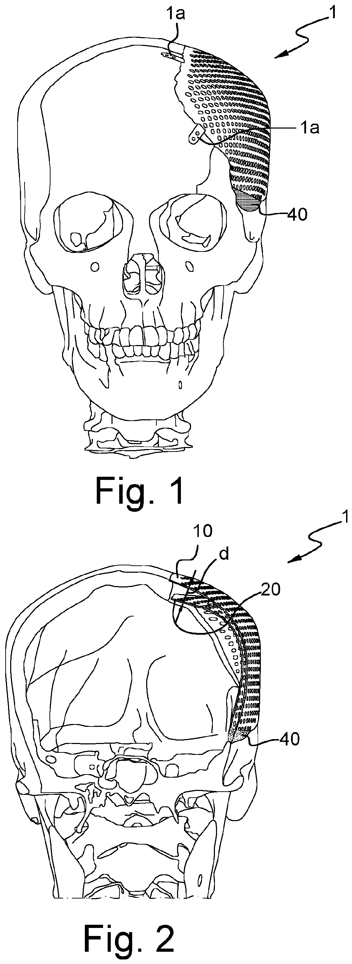

- figure 1 shows a computerized reconstruction of a front image of a cranial prosthesis according to the present invention implanted in the skull of a patient;
- figure 2 shows a section according to a front sectional plane of the implant of figure 1;
- figure 3 shows a rear sectional view with a front plane of the implant of figure 1;
- figure 4 shows an enlarged detail of the prosthesis according to the present invention;
- figure 5 shows an alternative embodiment of the cranial prosthesis according to the present invention;
- figure 6 shows a top view of a possible embodiment of the cranial prosthesis according to the present invention.

### DETAILED DESCRIPTION OF THE INVENTION

With particular reference to the attached figures, the cranial prosthesis 1 according to the present invention is configured to be implanted in a patient and is therefore made of biocompatible materials.

More specifically, the cranial prosthesis according to the invention is preferably made of titanium or a titanium alloy, and even more preferably is produced by means of additive manufacturing techniques for the sintering of powders, in particular with EBM (electron beam moulding) techniques.

Thanks to this particular production technology, it is possible to model the prosthesis with extreme precision on the patient's anatomy, detected by CAT (computerized axial tomography).

The cranial prosthesis **1** of biocompatible metal material according to the present invention is characterized in that it comprises a first plate **10** and a second plate **20.**

Advantageously, one or more spacer elements **30** are interposed between said first plate **10** and said second plate **20** and are suitable for keeping the two plates **10, 20** spaced apart.

Advantageously, said first plate **10** comprises a plurality of fixing holes **1a** positioned, for example, on sections of said first plate, which extend outwardly.

As already mentioned, each of the two plates **10, 20** is produced on the basis of the image acquired by CAT or similar methodology of the patient's bone to be restored.

Thanks to additive manufacturing techniques, it is therefore possible to exactly replicate the development of the bone surface to be restored using the prosthesis.

Even more specifically, thanks to implementation by means of titanium powder deposition techniques, extremely complex geometries can be obtained which cannot be obtained by moulding. In particular, it is therefore possible to produce a prosthesis **1** that exactly reproduces the shape of the bone to be restored.

More particularly, the prosthesis according to the present invention comprises, as already mentioned, two plates, a first plate **10** being modelled on the patient's anatomy to replicate the development of the outer surface of the cranial bone to be restored, and a second plate **20** being modelled on the patient's anatomy to replicate the development of the internal surface of the cranial bone to be restored.

It is in particular the second plate **20** that, with the prosthesis installed, comes into contact with the dura mater for following as much as possible the patient's anatomy in order to prevent cavities that can favour the accumulation of tissue fluid from remaining between the dura mater and the second plate **20** of the prosthesis.

Furthermore, the perfect adherence of the inner plate **20** to the dura mater can allow a double advantage to be obtained: on the one hand, there is an excellent stability of the prosthesis **1** and its adherence to the dura mater avoids, as already mentioned, the risk of tissue liquid pockets forming that could jeopardize the success of the implant itself, on the other hand, it makes the suturing of the prosthesis to the dura mater itself unnecessary for the surgeon, thus greatly simplifying surgery procedures for the surgeon and making the operation less onerous for the patient.

Said first **10** and second **20** plate therefore define an interspace having a variable thickness **d.**

The prosthesis **1** according to the present invention therefore has a sandwich structure which also allows, in addition to the above-mentioned advantages relating to the possibility of modelling the two surfaces, the further advantage of having a lightened, lightweight and radiolucent structure to be obtained.

The plurality of holes **50** which are advantageously obtained on both plates **10, 20** also contribute to the lightening of the prosthesis and its radiolucency.

Still more preferably, one or more of said holes **50** are oval in shape, so as to facilitate the suturing operations if, even though the design of the prosthesis is such that this is not necessary, the surgeon prefers in any case to give some stitches.

Perfectly circular holes would require a perfect alignment of the plates to be used for suturing the prosthesis, whereas oval-shaped holes are easier to use.

According to a preferred embodiment of the present invention, the second plate **20** can have a smaller extension than the first plate **10.**

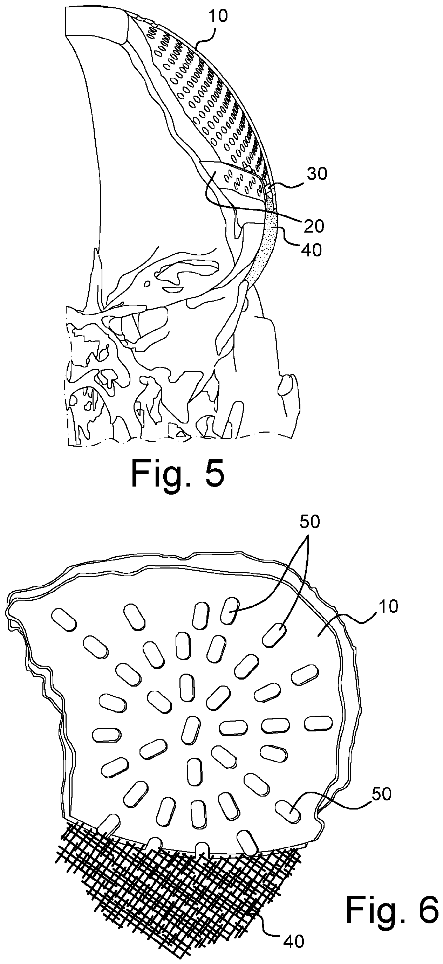

In this case, as shown in Figure 5, when the prosthesis **1** is implanted on the patient's skull, the second plate **20** is facing the inside of the skull, i.e. towards the dura mater, but does not affect the entire inner surface of the cranial bone to be restored.

Advantageously, and according to a preferred embodiment of the cranial prosthesis **1** according to the present invention, it further comprises at least one insert **40** having a trabeculated structure.

More specifically, the size of the trabeculae and the intratrabecular cavities are comparable to those of cancellous bone, so as to favour the osseous integration of the implant.

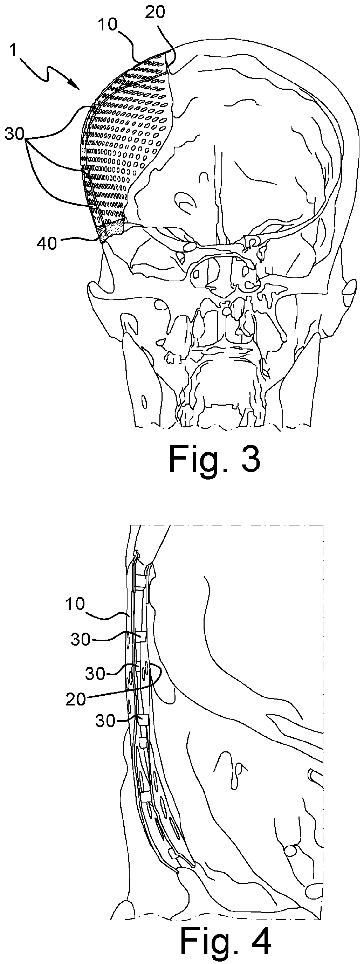

The insert **40** having a trabeculated structure preferably affects at least a portion of the perimeter of the prosthesis. In particular, according to what can be seen, for example, in Figures 3 and 5, said insert is positioned in correspondence with the temporal bone, where numerous muscles are grafted. It is therefore an advantage that is extremely appreciated by the surgeon to be able to shape this area of the prosthesis. The insert **40** with a trabeculated structure has the advantage of being able to be modelled by the surgeon during the operation using simple pincers, with which it is possible to shape the insert.

Said insert **40** having a trabeculated structure is preferably connected to both of the plates **10, 20** that form the prosthesis, in order to be stably connected to them. This situation can be seen, for example, in Figure 5, where, although a second plate **20** is provided which has a smaller extension with respect to the first plate **10,** the perimetric section of the prosthesis 1 involved by the insert **40** provides for the presence of both plates **10, 20** positioned adjacently to form a sandwich structure.

According to what has been described so far, the cranial prosthesis **1** according to the present invention is preferably made entirely of titanium or titanium alloys, and is produced by EBM techniques.

The Applicant has also verified that the particular configuration of the cranial prosthesis **1** according to the present invention, and in particular the possibility of modelling with precision on the patient's anatomy, both the first plate **10,** which, when the prosthesis is implanted, is facing the outside of the skull, and also the second plate **20,** which, when the prosthesis is implanted, is facing the inside of the skull, and also the possibility of providing, thanks to the sandwich structure, the presence of a mouldable insert **40** having a trabeculated form, offers the surgeon both the advantage of being able to avoid suturing the prosthesis to the dura mater during surgery, and also the advantage of being able to perfectly model the prosthesis in the temporal area, obtaining maximum stability and integration with the natural tissues.

The characteristics of the cranial prosthesis of the present invention, as also the relative advantages, are evident from the description so far provided.

It is also understood that the cranial prosthesis thus conceived can undergo numerous modifications and variations, all of which are included in the invention, the scope of which is defined by the claims.

In particular, the materials described, as also the dimensions, can vary according to technical requirements.

## Claims

1. A cranial prosthesis (1) made of biocompatible metal material for cranioplasty interventions comprising a first plate (10) and a second plate (20), one or more spacer elements (30) being interposed between said first plate (10) and said second plate (20), **characterized in that** said first plate (10) is modelled on the patient's anatomy to replicate the development of the outer surface of the cranial bone to be restored, and said second plate (20) is modelled on the patient's anatomy for replicate the development of the internal surface of the cranial bone to be restored , wherein said first (10) and second (20) plate define an interspace with a variable thickness (d).

2. The cranial prosthesis (1) according to the previous claim, wherein said second plate (20) has a smaller extension with respect to said first plate (10) and does not affect the entire inner surface of the cranial bone to be restored.

3. The cranial prosthesis (1) according to one or more of the previous claims, wherein it further comprises at least one mouldable insert (40) having a trabeculated structure.

4. The cranial prosthesis (1) according to the previous claim, wherein said mouldable insert (40) is positioned in correspondence with at least one perimetral section of the prosthesis (1).

5. The cranial prosthesis (1) according to one or more of the previous claims, wherein said first (10) and second plate (20) are provided with a plurality of holes (50).

6. The cranial prosthesis (1) according to the previous claim, wherein one or more of said holes (50) are oval in shape.

7. The cranial prosthesis (1) according to one or more of the previous claims, wherein it is made of titanium or titanium alloys.

## Patentansprüche

1. Schädelprothese (1) aus biokompatiblem Metallmaterial für kranioplastische Eingriffe, welche eine erste Platte (10) und eine zweite Platte (20) umfasst, wobei ein oder mehrere Abstandselemente (30) zwischen der ersten Platte (10) und der zweiten Platte (20) angeordnet sind, **dadurch gekennzeichnet, dass** die erste Platte (10) der Anatomie eines Patienten nachgebildet ist, um die Entwicklung der äußeren Oberfläche des wiederherzustellenden Schädelknochens nachzubilden, und dass die zweite Platte (20) der Anatomie des Patienten nachgebildet ist, um die Entwicklung der inneren Oberfläche des wiederherzustellenden Schädelknochens nachzubilden, wobei die erste (10) und die zweite (20) Platte einen Zwischenraum mit einer variablen Dicke (d) definieren.

2. Schädelprothese (1) nach dem vorherigen Anspruch, wobei die zweite Platte (20) eine geringere Ausdehnung in Bezug auf die erste Platte (10) aufweist und nicht die gesamte Innenfläche des wiederherzustellenden Schädelknochens beeinflusst.

3. Schädelprothese (1) nach einem oder mehreren der vorherigen Ansprüche, wobei sie darüber hinaus mindestens einen formbaren Einsatz (40) mit einer trabekulierten Struktur umfasst.

4. Schädelprothese (1) nach dem vorherigen Anspruch, wobei der formbare Einsatz (40) in Übereinstimmung mit mindestens einem perimetralen Abschnitt der Prothese (1) positioniert ist.

5. Schädelprothese (1) nach einem oder mehreren der vorherigen Ansprüche, wobei die erste (10) und die zweite Platte (20) mit einer Vielzahl von Löchern (50) versehen sind.

6. Schädelprothese (1) nach dem vorherigen Anspruch, wobei eines oder mehrere der Löcher (50) eine ovale Form aufweisen.

7. Schädelprothese (1) nach einem oder mehreren der vorherigen Ansprüche, wobei sie aus Titan oder Titanlegierungen hergestellt ist.

## Revendications

1. Prothèse crânienne (1) réalisée en un matériau métallique biocompatible pour des interventions de cranioplastie, comprenant une première plaque (10) et une seconde plaque (20), et un ou plusieurs élément(s) d'espaceur (30) qui est/sont interposé(s) entre ladite première plaque (10) et ladite seconde plaque (20), **caractérisée en ce que** ladite première plaque (10) est modélisée sur l'anatomie du patient pour répliquer le développement de la surface externe de l'os crânien à restaurer, et ladite seconde plaque (20) est modélisée sur l'anatomie du patient pour répliquer le développement de la surface interne de l'os crânien à restaurer, dans laquelle lesdites première (10) et seconde (20) plaques définissent un inter-espace présentant une épaisseur variable (d).

2. Prothèse crânienne (1) selon la revendication précédente, dans laquelle ladite seconde plaque (20) présente une extension plus petite par rapport à ladite première plaque (10) et n'affecte pas la totalité de la surface interne de l'os crânien à restaurer.

3. Prothèse crânienne (1) selon une ou plusieurs des revendications précédentes, dans laquelle elle comprend en outre au moins un insert moulable (40) qui présente une structure trabéculée.

4. Prothèse crânienne (1) selon la revendication précédente, dans laquelle ledit insert moulable (40) est positionné en correspondance avec au moins une section périmétrique de la prothèse (1).

5. Prothèse crânienne (1) selon une ou plusieurs des revendications précédentes, dans laquelle lesdites première (10) et seconde (20) plaques sont munies d'une pluralité de trous (50).

6. Prothèse crânienne (1) selon la revendication précédente, dans laquelle un ou plusieurs desdits trous (50) présente(nt) une forme ovale.

7. Prothèse crânienne (1) selon une ou plusieurs des revendications précédentes, dans laquelle elle est réalisée en titane ou en alliages de titane.
